(19) Europäisches Patentamt
European Patent Office
Office européen des brevets

(11) Veröffentlichungsnummer: **0 010 154**
**A1**

(12) # EUROPÄISCHE PATENTANMELDUNG

(21) Anmeldenummer: **79103384.8**

(22) Anmeldetag: **11.09.79**

(51) Int. Cl.$^3$: **A 01 N 53/00**
//C07C69/743

(30) Priorität: **21.09.78 DE 2840992**

(43) Veröffentlichungstag der Anmeldung:
**30.04.80 Patentblatt 80/9**

(84) Benannte Vertragsstaaten:
**AT BE CH DE FR GB IT NL SE**

(71) Anmelder: **BAYER Aktiengesellschaft**
**Zentralbereich Patente,Marken und Lizenzen**
**Bayerwerk**
**D-5090 Leverkusen 1(DE)**

(72) Erfinder: **Naumann, Klaus, Dr.**
**Wolfskaul 2**
**D-5000 Köln 80(DE)**

(72) Erfinder: **Klauke, Erich, Dr.**
**Eichendorffweg 8**
**D-5068 Odenthal(DE)**

(72) Erfinder: **Marhold, Albrecht, Dr.**
**Carl-Duisberg-Strasse 329**
**D-5090 Leverkusen 1(DE)**

(72) Erfinder: **Homeyer, Bernhard, Dr.**
**Obere Strasse 28**
**D-5090 Leverkusen 3(DE)**

(54) **Bodeninsektizide Mittel mit einem Gehalt an substituierten Cyclopropancarbonsäurebenzylestern (I) als Wirkstoff, Herstellung dieser Mittel, Bekämpfung von Bodeninsekten durch die Ester (I) und substituierte Cyclopropancarbonsäurebenzylester.**

(57) Die teilweise bekannten substituierten Cyclopropancarbonsäurebenzylester der allgemeinen Formel I

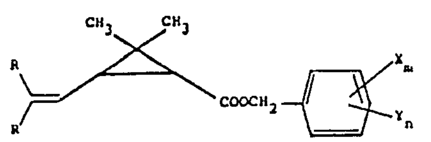

(I)

in welcher R für $CH_3$, Cl, Br oder F; X für Cl, Br oder J; Y für Cl oder F; und m und n für ganze Zahlen von 0 bis 5 stehen, unter der Voraussetzung, daß m und n nicht gleichzeitig 0 sein können, eignen sich zur Bekämpfung von Bodeninsekten.

Die neuen unter den Verbindungen der allgemeinen Formel I können durch Umsetzung eines entsprechenden reaktiven Säurederivats mit dem entsprechenden Alkohol oder seinem reaktiven Derivat hergestellt werden.

EP 0 010 154 A1

BAYER AKTIENGESELLSCHAFT 5090 Leverkusen, Bayerwerk
Zentralbereich
Patente, Marken und Lizenzen Rt/Th/Kü
II b

BEZEICHNUNG GEÄNDERT
siehe Titelseite

Mittel gegen Bodeninsekten

Die vorliegende Erfindung betrifft die Verwendung teilweise bekannter Cyclopropancarbonsäureester als Bodeninsektizide.

Es ist bereits bekannt geworden, daß Ester von 2,2-Dimethyl-(3-substituiertes vinyl)-cyclopropancarbonsäuren mit substituierten Benzylalkoholen insektizide Eigenschaften aufweisen. (Pestic. Sci. 1975, S. 537 - 542; Agr. Biol. Chem. 40, S. 247 - 249 (1976); FR-PS 2 290 415, FR-PS 2 271 196; DE-OS 2 658 074).

Von den eingehend untersuchten, interessanten Pyrethroiden ist bekannt geworden, daß sie als Bodeninsektzide nicht geeignet sind (Pestic. Sci. 1977, S. 241, Pestic. Sci. 1977 S. 267, Proceeding 1977 Brit. Crop. Protect. Conf. Band 2, S. 647 und 655). Daraus wurde geschlossen, daß Pyrethroide allgemein als Bodeninsektizide nicht geeignet seien (Pestic. Sci. 1977, S. 267; Nachr. Chem. Techn. Lab. 26 (1978) Nr. 3 S. 121).

Es wurde nun gefunden, daß die substituierten Cyclopropancarbonsäureester der allgemeinen Formel I

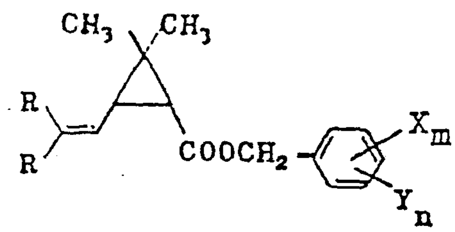

(I)

in welcher

R für $CH_3$, Cl, Br, F und

X für Cl, Br, J und

Y für Cl, F steht und

m und n für ganze Zahlen von 0 bis 5 stehen können, unter der Voraussetzung, daß m und n nicht gleichzeitig 0 sein können,

hochwirksame Bodeninsektizide sind.

Dieser überraschende Befund, welcher nach dem bisherigen Wissen nicht erwartet werden konnte, stellt somit eine wertvolle Bereicherung der Technik dar.

Die substituierten Cyclopropancarbonsäureester der Formel I sind zum Teil bekannt aus DE-OS 2 658 074 und FR-PS 2 271 196 und FR-PS 2 290 415.

Die substituierten Cyclopropancarbonsäureester der Formel I

in welcher
R die oben angegebene Bedeutung hat und
X für Br, J und
Y für Cl, F und
m für eine ganze Zahl von 1 bis 3 und
n für eine ganze Zahl von 0 bis 4 stehen
sind neu.

Sie werden hergestellt, indem man

a) ein Säurechlorid der Formel II

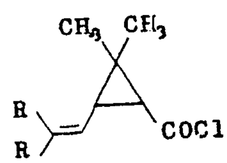

II

in welcher
R die oben angegebene Bedeutung hat,

mit einem Alkohol der Formel III

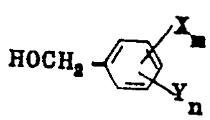

III

in welcher
X, Y, m und n die oben angegebene Bedeutung haben,

bei Temperaturen zwischen 20 und 100°C gegebenenfalls in Gegenwart eines Verdünnungsmittels oder eines Säurefängers umsetzt, oder indem man

b) ein Salz der allgemeinen Formel IV

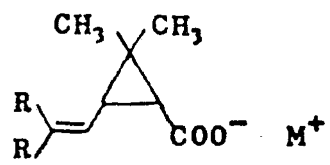

IV

in welcher

R die oben angegebene Bedeutung hat und

$M^+$ ein Alkali oder Erdalkalikation, oder ein primäres, sekundäres oder tertiäres Ammoniumion bedeutet,

mit einem Benzylhalogenid der allgemeinen Formel V

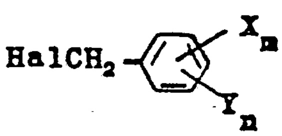

V

in welcher

Hal für Cl, Br steht und

X, Y, m und n die oben angegebene Bedeutung haben,

in einem polaren Lösungsmittel bei Temperaturen zwischen 20 und 120° umsetzt.

Die erfindungsgemäß verwendbaren Stoffe sind durch die Formel I allgemein definiert. Darin ist R vorzugsweise gleich oder verschieden und steht für Methyl, F bzw. Chlor oder Brom, insbesondere für Chlor oder Brom, m steht vorzugsweise für ganze Zahlen von 0 bis 5, n steht vorzugsweise für ganze Zahlen von 0 bis 5.

Die Verbindungen kommen in Form ihrer Stereoisomeren vor. Die Substituenten am Cyclopropanring können cis oder trans zueinander stehen. Die Kohlenstoffatome $C_1$ und $C_3$ können jeweils die R und/oder S-Konfiguration aufweisen und somit zu optisch aktiven bzw. racemischen cis und/ oder trans-Isomeren führen.

Als Beispiele für die erfindungsgemäß verwendbaren Stoffe seien genannt:
2,2-Dimethyl-3-dichlorvinylcyclopropancarbonsäure-pentafluorbenzylester
2,2-Dimethyl-3-dichlorvinylcyclopropancarbonsäure-tetrafluor-2-chlor-benzylester
2,2-Dimethyl-3-dichlorvinylcyclopropancarbonsäure-tetrafluor-3-chlor-benzylester
2,2-Dimethyl-3-dichlorvinylcyclopropancarbonsäure-tetrafluor-4-chlor-benzylester
2,2-Dimethyl-3-dichlorvinylcyclopropancarbonsäure-tetrafluor-3-brom-benzylester
2,2-Dimethyl-3-dichlorvinylcyclopropancarbonsäure-2,4,6-trifluor-dichlor-benzylester
2,2-Dimethyl-3-dichlorvinylcyclopropancarbonsäure-2,3,6-trifluor-dichlor-benzylester
2,2-Dimethyl-3-dichlorvinylcyclopropancarbonsäure-2,4,5-trifluor-dichlor-benzylester
2,2-Dimethyl-3-dichlorvinylcyclopropancarbonsäure-3,4,5-trifluor-dichlor-benzylester

2,2-Dimethyl-3-dichlorvinylcyclopropancarbonsäure-2,4,6-trifluor-dibrom-benzylester
2,2-Dimethyl-3-dichlorvinylcyclopropancarbonsäure-2,4,6-trichlor-difluor-benzylester
2,2-Dimethyl-3-dichlorvinylcyclopropancarbonsäure-2,3,6-trichlor-difluor-benzylester
2,2-Dimethyl-3-dichlorvinylcyclopropancarbonsäure-2,4,5-trichlor-difluor-benzylester
2,2-Dimethyl-3-dichlorvinylcyclopropancarbonsäure-3,4,5-trichlor-difluor-benzylester
2,2-Dimethyl-3-dichlorvinylcyclopropancarbonsäure-tetrachlor-2-fluor-benzylester
2,2-Dimethyl-3-dichlorvinylcyclopropancarbonsäure-tetrachlor-3-fluor-benzylester
2,2-Dimethyl-3-dichlorvinylcyclopropancarbonsäure-tetrachlor-4-fluor-benzylester
2,2-Dimethyl-3-dichlorvinylcyclopropancarbonsäure-2,3,5,6-tetrafluorbenzylester
2,2-Dimethyl-3-dichlorvinylcyclopropancarbonsäure-2,3,4,5-tetrafluorbenzylester
2,2-Dimethyl-3-dichlorvinylcyclopropancarbonsäure-2,3,5,6-tetrachlorbenzylester
2,2-Dimethyl-3-dichlorvinylcyclopropancarbonsäure-2,3,4,5-tetrachlorbenzylester
2,2-Dimethyl-3-dichlorvinylcyclopropancarbonsäure-2,4,6-trifluorbenzylester
2,2-Dimethyl-3-dichlorvinylcyclopropancarbonsäure-2,3,6-trifluorbenzylester
2,2-Dimethyl-3-dichlorvinylcyclopropancarbonsäure-2,4,5-trifluorbenzylester
2,2-Dimethyl-3-dichlorvinylcyclopropancarbonsäure-3,4,5-trifluorbenzylester
2,2-Dimethyl-3-dichlorvinylcyclopropancarbonsäure-2,4,6-trichlorbenzylester

2,2-Dimethyl-3-dichlorvinylcyclopropancarbonsäure-2,3,6-trichlorbenzylester
2,2-Dimethyl-3-dichlorvinylcyclopropancarbonsäure-2,4,5-trichlorbenzylester
2,2-Dimethyl-3-dichlorvinylcyclopropancarbonsäure-3,4,5-trichlorbenzylester
2,2-Dimethyl-3-dichlorvinylcyclopropancarbonsäure-2,4,6-trichlor-3-brombenzylester
2,2-Dimethyl-3-dichlorvinylcyclopropancarbonsäure-2,4,6-trichlor-3-jodbenzylester
2,2-Dimethyl-3-dichlorvinylcyclopropancarbonsäure-2,4-dichlor-6-brombenzylester
2,2-Dimethyl-3-dichlorvinylcyclopropancarbonsäure-3,5-dichlor-4-brombenzylester
2,2-Dimethyl-3-dichlorvinylcyclopropancarbonsäure-2,4-dichlor-5-jodbenzylester
2,2-Dimethyl-3-dichlorvinylcyclopropancarbonsäure-2,4-dichlorbenzylester
2,2-Dimethyl-3-dichlorvinylcyclopropancarbonsäure-3,4-dichlorbenzylester
2,2-Dimethyl-3-dichlorvinylcyclopropancarbonsäure-3,5-dichlorbenzylester
2,2-Dimethyl-3-dichlorvinylcyclopropancarbonsäure-2,4,6-tribrombenzylester
2,2-Dimethyl-3-dichlorvinylcyclopropancarbonsäure-2,4,5-tribrombenzylester
2,2-Dimethyl-3-dichlorvinylcyclopropancarbonsäure-2,4-dibrom-6-chlorbenzylester
2,2-Dimethyl-3-dichlorvinylcyclopropancarbonsäure-2,4-dibrom-benzylester
2,2-Dimethyl-3-dichlorvinylcyclopropancarbonsäure-3,4-dibrom-benzylester
2,2-Dimethyl-3-dichlorvinylcyclopropancarbonsäure-3,5-dibrom-benzylester

2,2-Dimethyl-3-dichlorvinylcyclopropancarbonsäure-2,6-dibrom-benzylester
2,2-Dimethyl-3-dichlorvinylcyclopropancarbonsäure-2,4-difluor-benzylester
2,2-Dimethyl-3-dichlorvinylcyclopropancarbonsäure-3,5-difluor-benzylester
2,2-Dimethyl-3-dichlorvinylcyclopropancarbonsäure-2-fluor-3-jod-benzylester
2,2-Dimethyl-3-dichlorvinylcyclopropancarbonsäure-4-fluor-3-jod-benzylester
2,2-Dimethyl-3-dichlorvinylcyclopropancarbonsäure-4-chlor-2-jod-benzylester
2,2-Dimethyl-3-dichlorvinylcyclopropancarbonsäure-2,4-difluor-3-chlor-5-jod-benzylester
2,2-Dimethyl-3-dichlorvinylcyclopropancarbonsäure-2-brom-4-jod-benzylester
2,2-Dimethyl-3-dichlorvinylcyclopropancarbonsäure-3-brom-4-jod-benzylester
2,2-Dimethyl-3-dichlorvinylcyclopropancarbonsäure-3-brom-benzylester
2,2-Dimethyl-3-dichlorvinylcyclopropancarbonsäure-3-jod-4-chlor-benzylester
2,2-Dimethyl-3-dichlorvinylcyclopropancarbonsäure-3-jod-4-fluor-benzylester
2,2-Dimethyl-3-dichlorvinylcyclopropancarbonsäure-3-jod-benzylester
und die entsprechenden 2,2-Dimethyl-3-(2-methylbuten-1-yl)-cyclopropancarbonsäure- sowie 2,2-Dimethyl-3-dibromvinylcyclopropancarbonsäureester.

Die aufgeführten Ester beinhalten sowohl die racemischen als auch die optisch aktiven, sowie die cis- und/oder trans-Isomeren.

Verwendet man als Säurechlorid der Formel II 2,2-Dimethyl-3-dichlorvinylcyclopropancarbonsäurechlorid und als Alkohol der Formel III 3-Jodbenzylalkohol, kann der Reaktionsablauf bei Verfahren a durch folgendes Formelschema wiedergegeben werden:

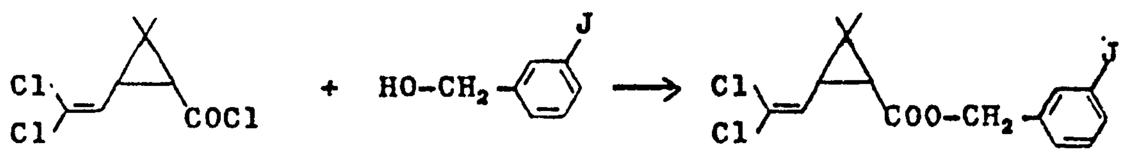

Die Verbindungen der allgemeinen Formel II sind bekannt (DE-OS 2 439 177).

Die Alkohole der allgemeinen Formel III sind teilweise bekannt (Beilstein, 5. Hauptband, S. 446, 447).

Neue Alkohole der Formel III lassen sich nach bekannten Verfahren darstellen, z.B. durch Reduktion der entsprechenden Carbonylverbindungen der allgemeinen Formel VIII

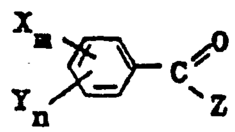

VIII

in welcher

X, Y, m und n die oben erwähnte Bedeutung haben und

Z für Hydroxy, Alkoxy, Chlor, Fluor oder Wasserstoff steht

mit komplexen Metallhydriden

Die für die Reduktion benötigten Carbonylverbindungen der Formel VIII sind bekannt. (Beilstein, 9. Hauptband, Syst. Nr. 938, S. 355-368; 9. Hauptband, 3- Erg. Werk Syst. Nr. 938, S. 1378, 1427 - 1431, S. 1452 - 1456).

Als Beispiele für die Alkohole der Formel III seien im einzelnen genannt:

2- bzw. 3- bzw. 4-Jodbenzylalkohol
2- bzw. 3- bzw. 4-Brombenzylalkohol
4-Fluor-4-jodbenzylalkohol
4-Chlor-3-jodbenzylalkohol
4-Brom-3-jodbenzylalkohol
Dichlor-brombenzylalkohol
Dichlor-jodbenzylalkohol
Dijod-chlorbenzylalkohol
Dibrom-chlorbenzylalkohol
Dibrom-benzylalkohol
Dijodbenzylalkohol.

Verwendet man als Salz der allgemeinen Formel IV Natrium-2,2-dimethyl-3-dichlorvinylcyclopropan-1-carboxylat und als Benzylhalogenid der allgemeinen Formel V 3,5-Dibrombenzylchlorid, läßt sich der Reaktionsablauf bei Verfahren b durch folgendes Formelschema wiedergegeben:

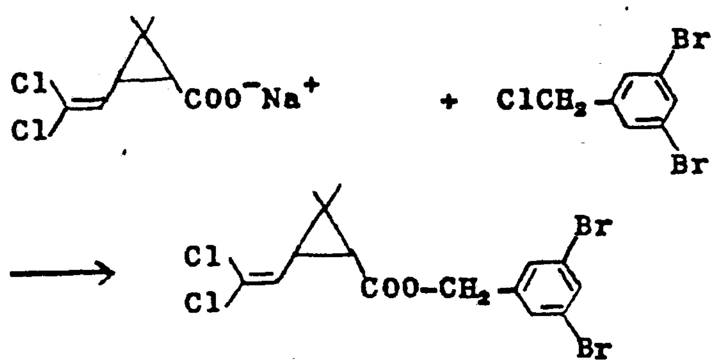

Die bei der Durchführung des Verfahrens b) als Ausgangsstoffe verwendbaren Salze der Formel IV sind bekannt (DE-OS 2 436 178). Die als Reaktionspartner benötigten Benzylhalogenide der Formel V sind teilweise bekannt (Beilstein, 5. Hauptband, 3. Ergänzungsband Syst. Nr. 466, S. 717-720, S. 726-727).

Neue Benzylchloride der allgemeinen Formel V können in an sich bekannter Weise durch Halogenierung der entsprechenden Toluole (Beilstein, 5. Hauptband, 3. Erg.-Band Syst. Nr. 466, S. 719-720, S. 725-728) hergestellt werden. Als Beispiele für die erfindungsgemäß verwendbaren Benzylchloride sei 3,5-Dibrombenzylchlorid erwähnt.

Das Verfahren gemäß b) wird in polaren organischen Verdünnungsmitteln wie Ketonen (z.B. Aceton), Nitrilen (z.B. Acetonitril), Säureamiden (z.B. DMF, Hexamethylphosphorsäuretriamid) oder ihren Gemischen mit Wasser durchgeführt. Es wird bei Temperaturen zwischen 20 und 100°C gearbeitet.

Es wird in Anwesenheit oder Abwesenheit von geeigneten Katalysatoren gearbeitet. Solche Katalysatoren sind peralkylierte Polyamine wie Pentamethyldiethylentriamin. Die Reaktionsdurchführung erfolgt wie in Synthesis 1975 S. 805 beschrieben.

Die Wirkstoffe eignen sich zur Bekämpfung von tierischen Schädlingen, insbesondere Insekten, welche im Boden leben. Sie sind gegen normal sensible und resistente Arten sowie gegen alle oder einzelne Entwicklungsstadien wirksam.

Die Wirkstoffe eignen sich bei guter Pflanzenverträglichkeit und günstiger Warmblütertoxizität zur Bekämpfung von tierischen Schädlingen, insbesondere Insekten, die in der Landwirtschaft vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophaus, Scutigera spec.
Aus der Ordnung der Symphyla z.B. Scutigerella immaculata.
Aus der Ordnung der Collembola z.B. Onychiurus armatus.
Aus der Ordnung der Orthoptera z.B. Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Linognathus spp.
Aus der Ordnung der Lepidoptera z.B. Agrotis spp., Euxoa spp., Feltia spp Earias insulana, Prodenia litura, Spodoptera spp., Chilo spp., Pyrausta nubilalis,
Aus der Ordnung der Coleoptera z.B. Diabrotica spp., Psylliodes chrysocephala, Atomaria spp., Sitophilus spp., Otiorrhynchus sulcatus, Cosmopolites sordidus, Ceuthorrhynchus assimilis, Tenebrio molitor, Agriotes spp., Conoderus spp., Melolontha melolontha, Amphimallon solstitialis, Costelytra zealandica.

Aus der Ordnung der Diptera z.B. Bibio hortulanus, Oscinella frit, Phorbia spp., Pegomyia hyoscyami, Tipula paludosa.

Die Wirkstoffe können in die üblichen Formulierungen übergeführt werden, wie Lösungen, Emulsionen, Spritzpulver, Suspensionen, Pulver, Stäubemittel, lösliche Pulver, Granulate, Köder, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methylethylketon, Methylisobutylketon oder Cyclohexanon,

stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Anwendung der erfindungsgemäßen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer den Anwendungsformen angepaßten üblichen Weise, in der Regel durch Spritzen, Streuen bzw. Stäuben auf oder in den Boden oder aber durch Verabreichung in Ködern, die beim Auftreten von Schädlingen ausgestreut werden.

Beispiel A

Grenzkonzentrations-Test /Bodeninsekten

Testinsekt: Tenebrio molitor-Larven im Boden
Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Wirkstoffe, Aufwandmengen und Resultate gehen aus der nachfolgenden Tabelle hervor:

Tabelle

Bodeninsektizide

Tenebrio molitor-Larven im Boden

| Wirkstoff (Konstitution) | Abtötungsgrad in % bei Wirkstoffkonzentration in ppm<br>2,5 ppm |
| --- | --- |
| bekannt Permethrin 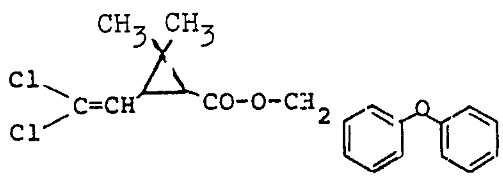 | 0 |
| Sumicidin 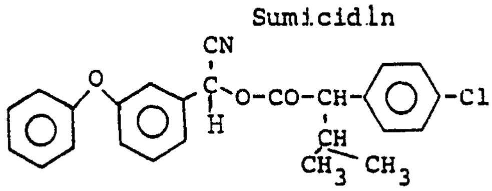 | 0 |
| 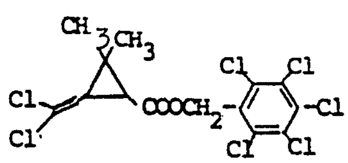 | 100 |
| 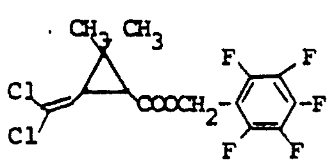 | 100 |

**Tabelle**

Bodeninsektizide

Tenebrio molitor-Larven im Boden

| Wirkstoff (Konstitution) | Abtötungsgrad in % bei Wirkstoffkonzentration in ppm 2,5 ppm |
|---|---|
| 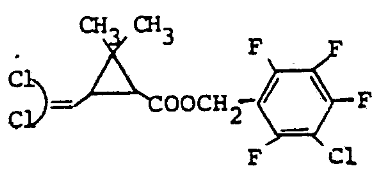 | 100 |
| 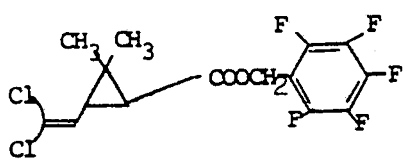 (±) trans-isomeres | 100 |
| 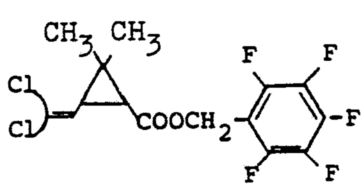 cis | 100 |
| 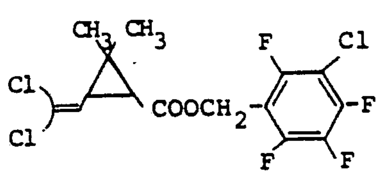 cis | 100 |

## T a b e l l e

Bodeninsektizide

Tenebrio molitor-Larven im Boden

| Wirkstoff (Konstitution) | Abtötungsgrad in % bei Wirkstoffkonzentration in ppm<br>2,5 ppm |
|---|---|
| 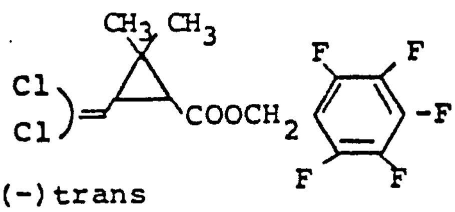<br>(-)trans | 100 |
| 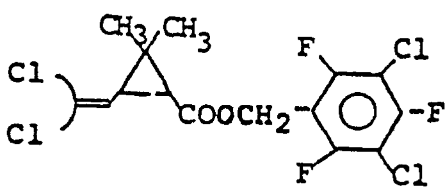 | 100 |

Beispiel B

Grenzkonzentrations-Test / Bodeninsekten

| | | | |
|---|---|---|---|
| Testinsekt: | Agrotis segetum-Larven im Boden | | |
| Lösungsmittel: | 3 | Gewichtsteile | Aceton |
| Emulgator: | 1 | Gewichtsteil | Alkylarylpolyglykol-äther |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (=mg/l) angegeben wird. Man füllt den Boden in Töpfe und läßt diese bei Raumtemperatur stehen.

Nach 24 Stunden werden die Testtiere in den behandelten Boden gegeben und nach weitern 2 bis 7 Tagen wird der Wirkungsgrad des Wirkstoffs durch Auszählen der toten und lebeneden Testinsekten in % bestimmt. Der Wirkungsgrad ist 100% wenn alle Testinsekten abgetötet worden sind, er ist 0% wenn nach genau so viele Testinsekten leben wie bei der unbehandelten Kontrolle.

Wirkstoffe, Aufwandmengen und Resultate gehen aus der nachfolgenden Tabelle hervor:

T a b e l l e

Bodeninsektizide

Agrotis segetum-Larven im Boden

| Wirkstoff (Konstitution) | Abtötungsgrad in % bei Wirkstoffkonzentration in ppm |
|---|---|
| | 0,5 ppm |
| bekannt Sumicidin 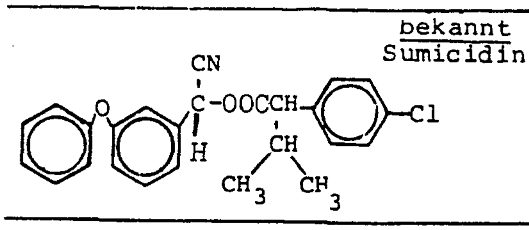 | 0 % |
| 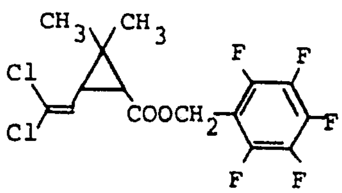 | 100 % |
| 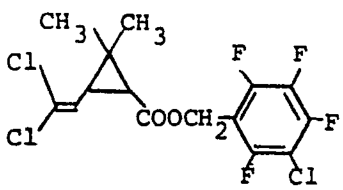 | 100 % |
| 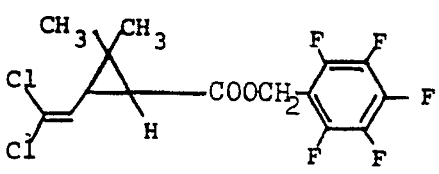 | 100 % |

**T a b e l l e**

Bodeninsektizide

Agrotis segetum-Larven im Boden

| Wirkstoff (Konstitutuion) | Abtötungsgrad in % bei Wirkstoffkonzrntration in ppm |
|---|---|
| | 0,5 ppm |
| 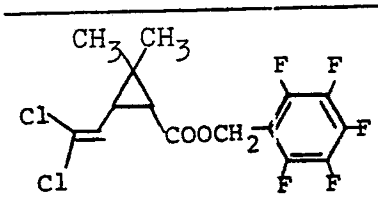 | 100 % |
| 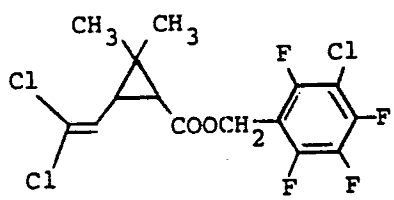 | 100 % |
| 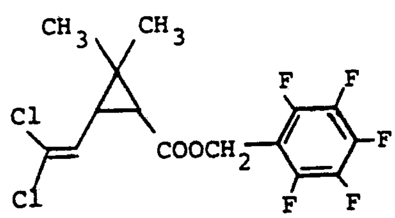 | 100 % |

Beispiel 1

2,2-Dimethyl-3-dichlorvinyl-cyclopropancarbonsäure-3-jodbenzylester

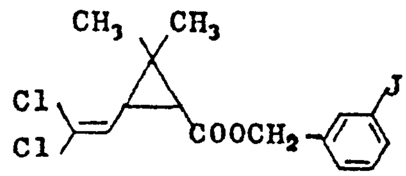

0,01 Mol 2,2-Dimethyl-3-dichlorvinylcyclopropancarbonsäurechlorid und 0,02 Mol 3-Jodbenzylalkohol wurden zusammen auf 100° erhitzt bis zum Ende der HCl-Entwicklung. Das nachbleibende Öl wurde dann am Kugelrohr bei 160° und 0,1 Torr destilliert.

IR: 2950, 1790, 1630, 1610, 1580, 1460, 1420, 1390, 1350, 1290, 1270, 1230, 1180, 1170, 1140, 1120, 1090, 1060, 1000, 930, 890, 870, 810, 785, 735, 700 $cm^{-1}$

Beispiel 2

Analog wurde der 3-Brom-benzylester hergestellt:

IR: 2950, 1720, 1610, 1580, 1450, 1410, 1390, 1350, 1270, 1230, 1180, 1110, 1090, 1060, 1000, 970, 930, 890, 860, 785, 700, 690 $cm^{-1}$.

Patentansprüche:

1. Bodeninsektizide Mittel gekennzeichnet durch einen Gehalt an substituierten Cyclopropancarbonsäureestern der allgemeinen Formel I

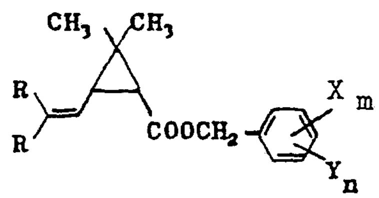

I

in welcher

R für $CH_3$, Cl, Br, F und

X für Cl, Br, J und

Y für Cl, F steht, und

m und n für ganze Zahlen von 0 bis 5 stehen können, unter der Voraussetzung, daß m und n nicht gleichzeitig 0 sein können.

2. Verwendung von substituierten Cyclopropancarbonsäureestern der Formel I zur Bekämpfung von Bodeninsekten.

3. Verfahren zur Bekämpfung von Bodeninsekten, dadurch gekennzeichnet, daß man substituierte Cyclopropancarbonsäureester der Formel I auf Bodeninsekten und/oder ihren Lebensraum einwirken läßt.

4. Verfahren zur Herstellung bodeninsektizider Mittel, dadurch gekennzeichnet, daß man substituierten Cyclopropancarbonsäureester der Formel I mit Streckmitteln und/oder oberflächenaktiven Mitteln vermischt.

5. Substituierten Cyclopropancarbonsäureester der Formel I

in welcher

R die oben angegebene Bedeutung hat
X für Brom oder Jod und
Y für Chlor oder Fluor und
m für eine ganze Zahl von 1 bis 3 und
n für eine ganze Zahl von 0 bis 4 steht.

Europäisches Patentamt

# EUROPÄISCHER RECHERCHENBERICHT

Nummer der Anmeldung 0010154

EP 79103384.8

| Kategorie | EINSCHLÄGIGE DOKUMENTE – Kennzeichnung des Dokuments mit Angabe, soweit erforderlich, der maßgeblichen Teile | betrifft Anspruch |
|---|---|---|
| P | DE - A1 - 2 721 185 (BAYER AG)<br>+ Ansprüche 1 - 4; Seite 14, letzter Absatz + | 5,<br>1 - 3 |
| | DE - A1 - 2 710 174 (BAYER AG)<br>+ Anspruch 1; Seite 4, 1. Absatz + | 5,<br>1 - 3 |
| | AT - B - 349 444 (SAGAMI CHEMICAL RESEARCH CENTER)<br>+ Seite 3, Zeilen 11 - 14 + | 1 - 3 |
| | DE - C - 1 150 242 (BENZOL PRODUCTS COMPANY) | 1 - 5 |
| | CH - A - 602 010 (CIBA-GEIGY AG) | 1 - 5 |
| | CH - A - 600 771 (CIBA-GEIGY AG) | 1 - 5 |
| | CH - A - 448 608 (NATIONAL RESEARCH DEVELOPMENT CORPORATION) | 1 - 5 |
| | GB - A - 1 116 206 (NATIONAL RESEARCH DEVELOPMENT CORPORATION) | 1 - 5 |

KLASSIFIKATION DER ANMELDUNG (Int.Cl. 3)

A 01 N 53/00 //
C 07 C 69/743

RECHERCHIERTE SACHGEBIETE (Int. Cl. 3)

A 01 N 53/00 //
C 07 C 69/00

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| WIEN | 30-11-1979 | HLAVA |